# EUROPEAN PATENT APPLICATION

(11) **EP 0 938 912 A1**
(43) Date of publication of application: **01.09.1999**
(21) Application number: 99650018.7
(22) Date of filing: 26.02.1999
(51) Int. Cl.: A61N 1/36

(54) **Muscle stimulation apparatus**

(30) Priority: 27.02.1998 IE 980146
(71) Applicant: BMR Research and Development Limited, County Galway (IE)
(72) Inventor: Keaney, Diarmuid, County Galway (IE)
(74) Representative: Brophy, David

(57) **Abstract**

A muscle stimulation apparatus comprises an operator's console (10) having a plurality of pairs of stimulation electrodes (32) and a stand (12) for removably accomodating the console. The stand (12) and console (10) have cooperating locating means which permit the console to be selectively located on the stand facing in either one of two opposite directions (Figs. 1 and 7). The console (10) comprises a display panel (44, Fig. 2) which can be folded down onto the top surface of the console from an upstanding in-use position.

## Description

This invention relates to a muscle stimulation apparatus.

According to the present invention there is provided a muscle stimulation apparatus comprising an operator's console having a plurality of pairs of stimulation electrodes connected thereto, and a stand for removably accomodating the console, the stand and console having cooperating locating means which permit the console to be selectively located on the stand facing in either one of two opposite directions.

There is also provided, as a further and independent invention, a muscle stimulation apparatus comprising an operator's console having a plurality of pairs of stimulation electrodes connected thereto, the console further comprising a display panel which can be folded down onto the top surface of the console from an upstanding in-use position.

An embodiment of the invention will now be described, by way of example, with reference to the accompanying drawings, in which:
Fig. 1 is a top plan view of a muscle stimulation apparatus;
Fig. 2 is a front view of the control console of Fig. 1 with the LCD display panel in an upright position;
Fig. 3 is a front view of the console with the LCD display panel folded down against the top surface of the console;
Fig. 4 is a top plan view of the console with the LCD display panel removed;
Figs. 5(a), 5(b) and 5(c) are rear, bottom and side views respectively of the lower housing part of the console;
Fig. 5(d) is a cross-section along the line A-A of Fig. 5(b);
Fig. 6 is a bottom plan view of the stand of Fig. 1; and
Fig. 7 is a top plan view of the muscle stimulation apparatus of Fig. 1 with the control console orientated in the opposite direction to Fig. 1.

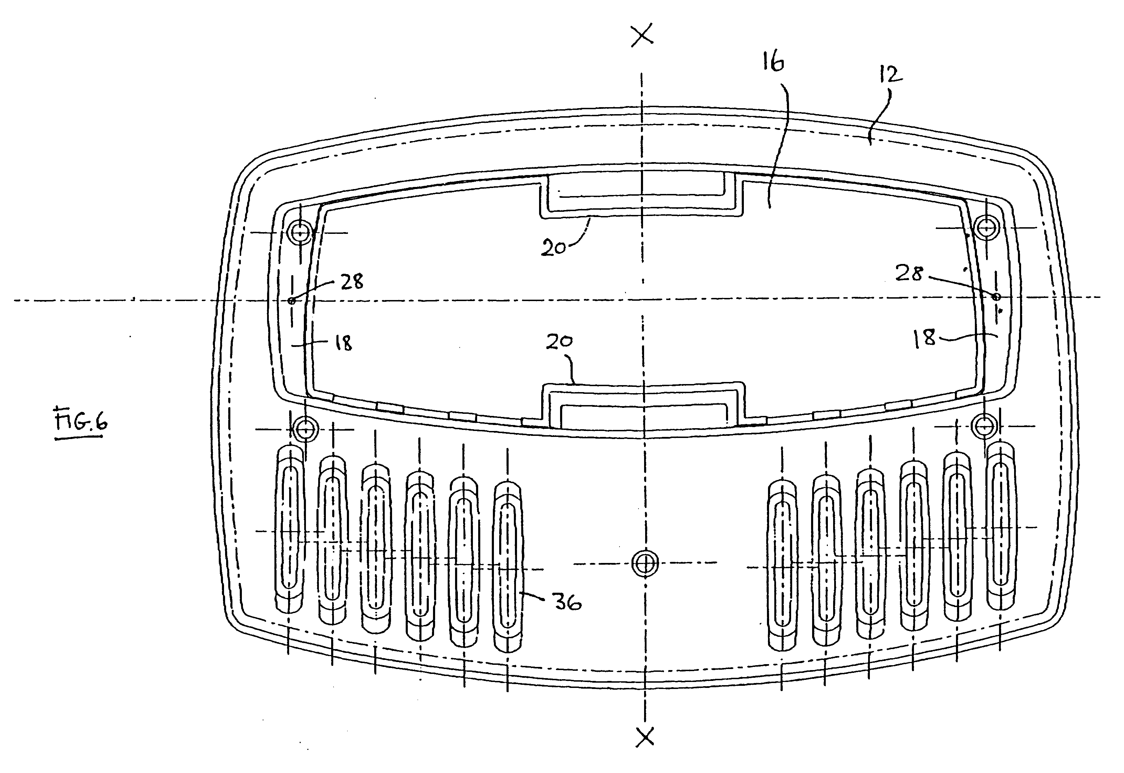

The apparatus comprises two main parts, a control console 10 and a stand 12, in the form of a storage tray. The stand has an elongate central aperture 16, Fig. 6, with a pair of horizontal shoulders or ledges 18, one at each narrow end of the aperture 16. The aperture 16 is of reduced width at its centre, as defined by slightly concave opposing surfaces 20.

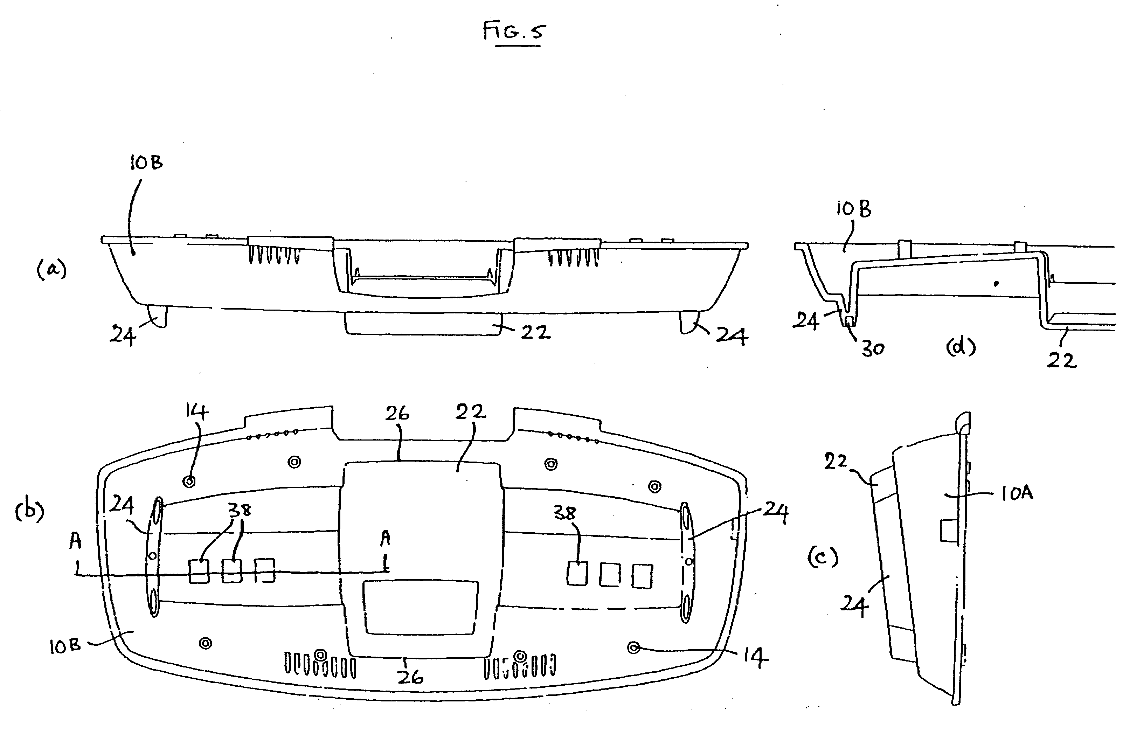

The console 10 is constructed in two parts, an upper housing 10A and a lower housing 10B which are secured together by screws at the points 14, Fig. 5(b). The lower surface of the lower housing 10B has a relatively large downwardly extending protuberance 22 at its centre and a pair of downwardly extending flanges 24, one on either side of the protuberance 22 near the narrow ends of the lower housing 10B. The opposite walls 26 of the protuberance 22 are slightly convex, to the same degree as the surfaces 20 are concave, and they are spaced apart by substantially the same distance as the surfaces 20 so that the protuberance 22 is a snug fit between the surfaces 20.

The console 10 is accommodated on the stand 12 by locating the protuberance 22 between the opposite surfaces 20 of the aperture 16 in the stand and allowing the console 10 to slide downwardly. The flanges 24 are spaced apart by approximately the same distance as the ledges 18, so that ultimately each flange 24 will come to rest on a respective ledge 18. The console 10 is secured in this position by screws which pass through the tray at the points 28 and enter the flanges 24 at the points 30, Fig. 5(d).

The console 10 has twelve pairs of stimulation electrodes 32 connected thereto by respective twin core leads 34. The pairs of electrodes 32, which are approximately circular, are accomodated by their edges in respective slots 36 in the top surface of the stand 12 when not in use. The leads 34 are connected to the internal circuitry of the console 10 via connectors 38 on the underside of the console, Fig. 5(b), two leads 34 being connected to each connector 38.

For each pair of electrodes 32 there is a corresponding intensity control 40 for adjusting the intensity of the stimulation signal supplied by that pair of electrodes. Each control 40 comprises an increment button 40A, a decrement button 40B and a row of LEDs 42. Pressing the increment button 40A and holding it pressed will gradually increase the signal intensity, while pressing the decrement button 40B and holding it pressed will gradually decrease the signal intensity. The instantaneous intensity is indicated to the user by the LEDs 42; consecutively more are lit the greater the intensity and vice versa.

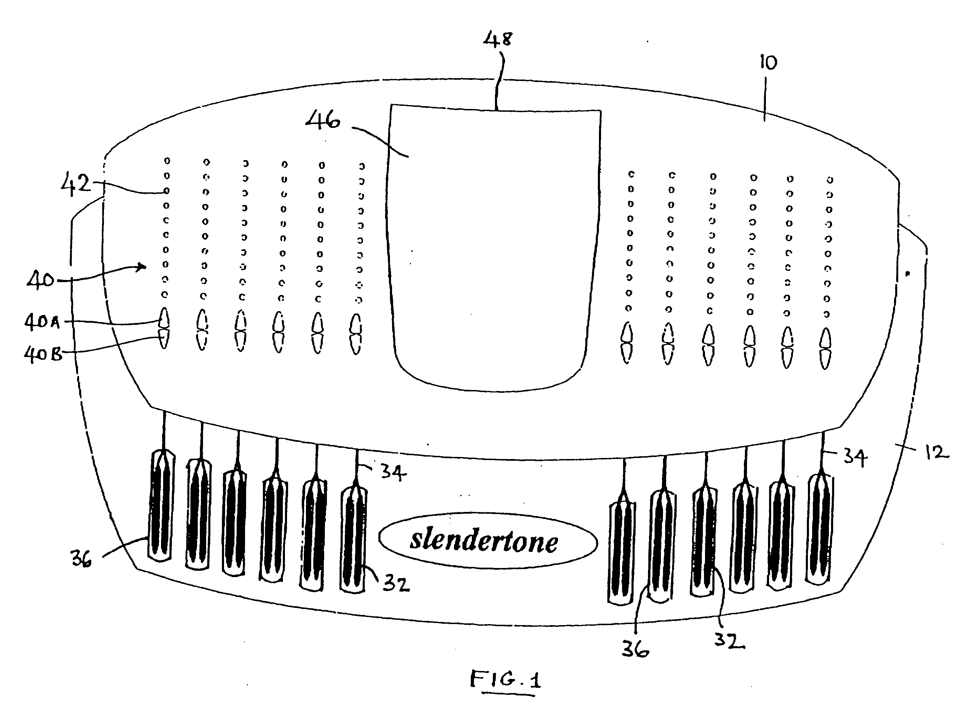

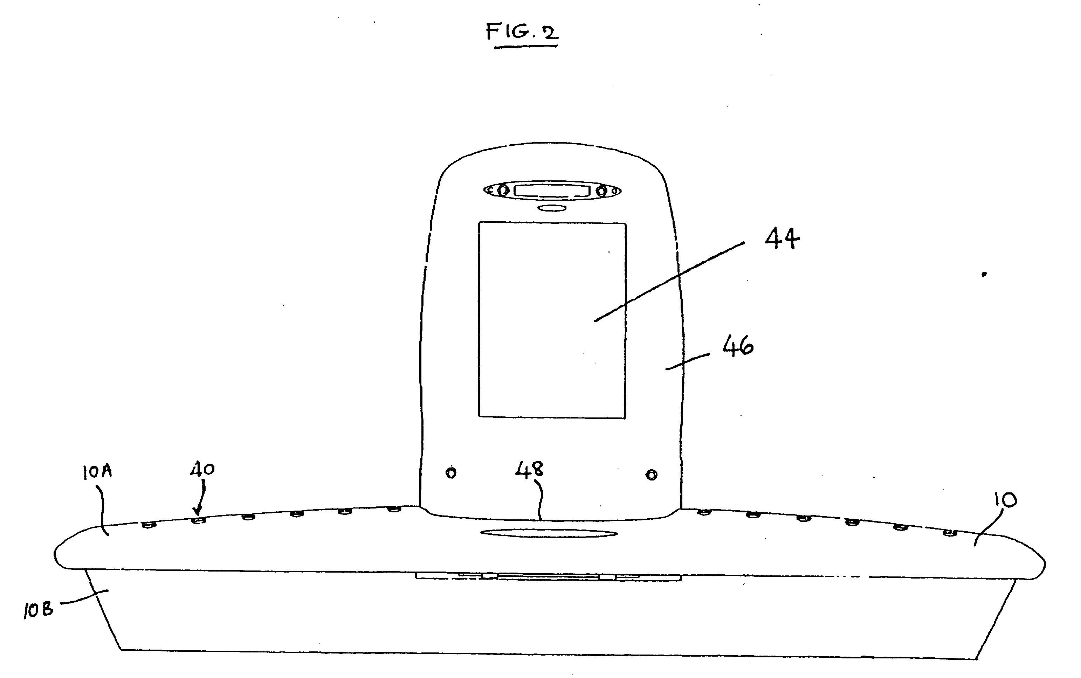

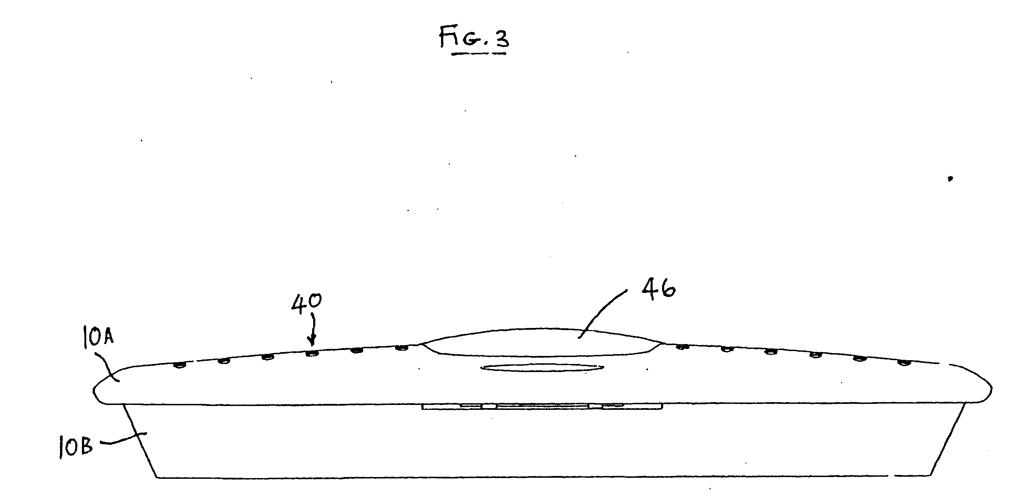

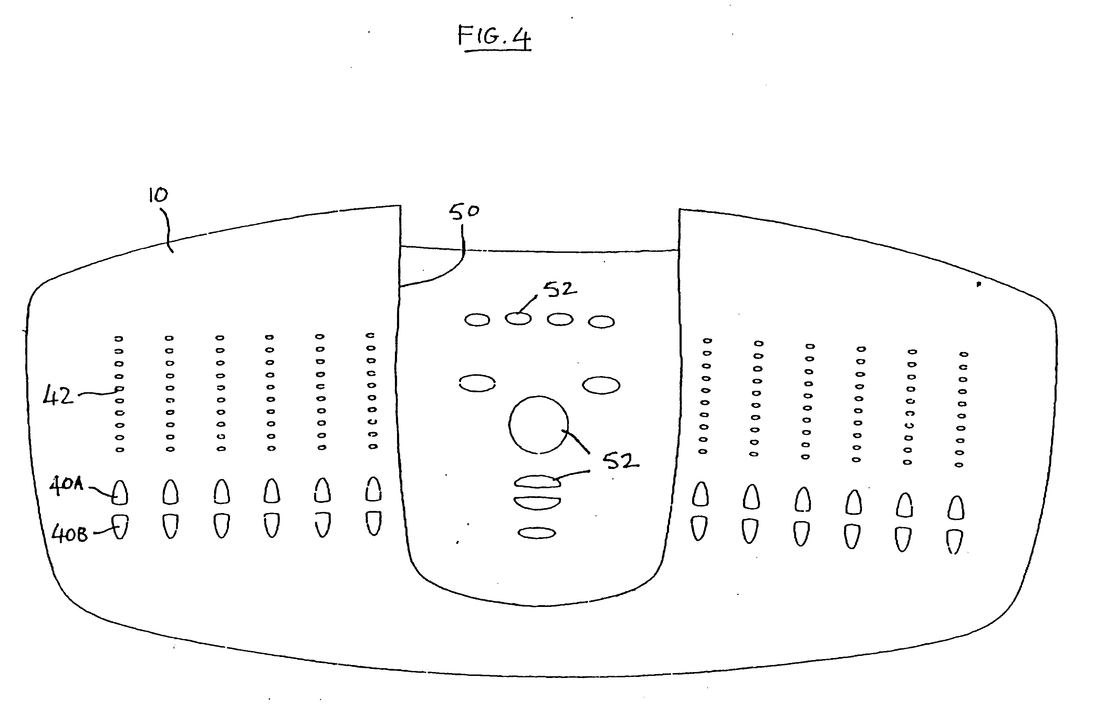

The console 10 further includes an LCD display panel 44, Fig. 2. This is mounted in a housing or lid 46 which is hinged along one edge 48 to the main body of the console. When the apparatus is in use the housing 46 stands in an upright position, Fig. 2, so that the LCD display may be viewed by the operator. However, when the apparatus is not in use the housing 46 and display panel 44 may be folded down onto the top surface of the console 10, Figs. 1 and 3, and is received snugly in a recess 50, Fig. 4, in the top surface of the console 10.

The recess 50 contains various buttons 52 for selecting different treatment regimes for the patient which are programmed into the console 10. Choosing a given treatment regime selects the operating frequency for the different electrodes 32, and illustrates on the display panel 44 where each electrode is to be located on the patient's skin for that regime. However, the intensity of the stimulation signals has to be set by the intensity controls 40.

For ease of use, the pairs of electrodes 32 and the corresponding intensity controls are colour coded. Thus, in this embodiment, each electrode pair 32 has a lead 34 whose colour is different at least from its immediate neighbour(s), and the control buttons 40A, 40B which control the signal intensity of each electode pair 32 is coloured the same as the respective lead.

Fig. 1 shows the console 10 oriented on the stand 12 such that when the lid 46 is opened the LCD display panel 44 faces the front of the apparatus (the lowermost edge of the stand as seen in Fig. 1). This would be most appropriate when both the patient and operator are seated in front of the apparatus, or if the patient is self-administering the treatment.

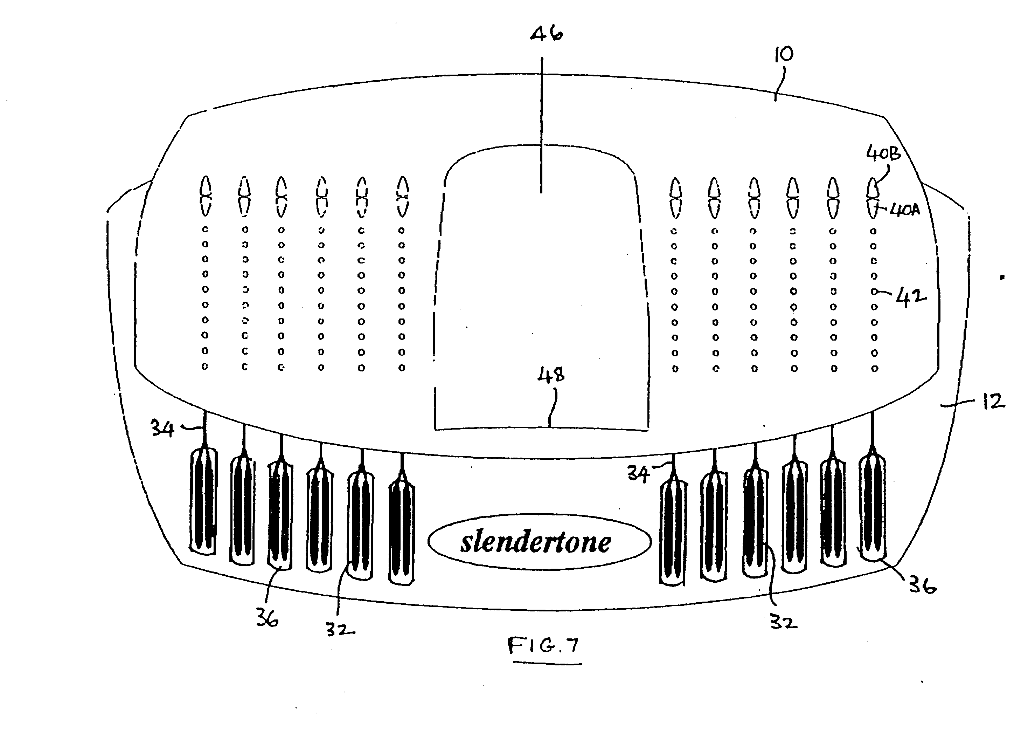

However, in some cases the operator may wish to sit on the opposite side of the apparatus to the patient, and may wish the display panel 44 to face the rear of the apparatus. In this case, after releasing the screws at points 28, the console 10 may be removed from the stand 12, turned through 180 degrees, and re-inserted in the stand 12, after which the screws are replaced. This opposite orientation is shown in Fig. 7.

To facilitate this, the opposite walls 26 of the protuberance 22 and the opposite surfaces 20 of the aperture 16 are symmetrical about the minor axis X-X, Fig. 6, of the apparatus, so that the protuberance 22 may fit snugly either way between the surfaces 20. Further, the flanges 24 are designed to come to rest on the ledges 18 whichever way round the protuberance 22 is fitted between the surfaces 20.

The invention is not limited to the embodiments described herein which may be modified or varied without departing from the scope of the invention.

## Claims

1. A muscle stimulation apparatus comprising an operator's console having a plurality of pairs of stimulation electrodes connected thereto, and a stand for removably accomodating the console, the stand and console having cooperating locating means which permit the console to be selectively located on the stand facing in either one of two opposite directions.

2. An apparatus as claimed in claim 1, whereing the console includes a respective control means associated with each electrode pair for adjusting the intensity of the stimulation signal supplied thereby, and means for colour coding each electrode pair and its respective control means.

3. An apparatus as claimed in claim 2, wherein each control means includes a plurality of lights, wherein the number of lights lit increases with increasing intensity.

4. An apparatus as claimed in claim 1, 2 or 3, the console further comprising a display panel which can be folded down onto the top surface of the console from an upstanding in-use position.

5. An apparatus as claimed in claim 1, wherein the tray has means for removably accomodating the pairs of electrodes.

6. A muscle stimulation apparatus comprising an operator's console having a plurality of pairs of stimulation electrodes connected thereto, the console further comprising a display panel which can be folded down onto the top surface of the console from an upstanding in-use position.
